# EUROPEAN PATENT APPLICATION

(11) **EP 1 857 149 A1**
(43) Date of publication of application: **21.11.2007**
(21) Application number: 07009446.1
(22) Date of filing: 10.05.2007
(51) Int. Cl.: A63B 23/03

(54) **Assisting device for practicing lateral movement of lower jaw**

(30) Priority: 18.05.2006 JP 2006138777
(71) Applicant: Miyama, Masaru, Asaminami-ku Hiroshima City Hiroshima 731-0141 (JP)
(72) Inventor: Miyama, Masaru, Asaminami-ku Hiroshima City Hiroshima 731-0141 (JP)
(74) Representative: Rupp, Christian

(57) **Abstract**

There is provided an assisting device for practicing lateral movement of a lower jaw to move a cheek-side cusp of a lower molar to a cheek-side surface of an upper molar. The assisting device includes an occlusion portion, which is placed in an oral cavity of a user and occluded by an upper central incisor and a lower central incisor, and a handle connected with the occlusion portion. The occlusion portion includes an upper tooth contact surface to be in contact with an incisal edge of the upper central incisor, and a lower tooth contact surface to be in contact with an incisal edge of the lower central incisor. The lower tooth contact surface has a flat surface. The occlusion portion provides a space between the incisal edge of the upper central incisor and the incisal edge of the lower central incisor. The upper tooth contact surface and the lower tooth contact surface cover entire movement ranges of the incisal edge of the upper central incisor and the incisal edge of the lower central incisor, respectively, when the lateral movement of the lower jaw is being practiced. It is, therefore, possible to provide an assisting device for practicing lateral movement of a lower jaw to assist a user to laterally move the lower jaw in a smooth manner.

## Description

### CROSS-REFERENCE TO RELATED APPLICATION(S)

This application claims the foreign priority benefit under Title 35, United States Code, §119 (a)-(d), of Japanese Patent Application No. 2006-138777 filed on May 18, 2006 in the Japan Patent Office, the disclosure of which is herein incorporated by reference in its entirety.

### BACKGROUND OF THE INVENTION

### 1. Field of the Invention

The present invention relates to an assisting device for practicing lateral movement of a lower jaw to improve systemic disorders due to the difficulty of the lateral movement of a lower jaw.

### 2. Description of the Related Art

It is said that keeping one's own teeth for a lifetime is one of conditions to live a happy life. It is also necessary to be healthy without having any systemic diseases in order to live happily.

Although most people apparently live a happy life, some of them suffer from pain, disorder, or discomfort symptoms (referred to as a systemic disorder) of unknown causes and they do not think it possible to cure such a systemic disorder. Others do not know causes or medical treatments of systemic disorders and live with such systemic disorders, which ruin their lives.

According to recent studies, it has become clear that such systemic disorders of unknown causes are associated with teeth, especially with the function of the teeth (jaw), that is how the teeth (jaw) move, rather than the structure of the teeth, that is how many teeth remain.

Jaw movement of a healthy individual is explained by three-dimensional movement. In specific, the vertical movement is described by a centric occlusion, a centric relation, and a rest position, and the horizontal movement is described by a gothic arch or the like. It is known that, a problem with the jaw movement causes a serious problem of temporomandibular joints, which could lead to discomfort symptoms around the jaw and in mentality of a person.

Wit regard to the systemic disorder and a problem of the jaw and the oral cavity, the systemic disorder in association with a problem of the temporomandibular joints is known, as disclosed by Hirokazu Nakamura, "Clinicostatistical study on patients of dentistry and oral surgery treated with anti-depressants", Japanese Journal of Psychosomatic Dentistry, 1989, volume 4, No. 1, pages 8-16, and by Masaru Miyama "A Case Report of Treatment of TMJ Arthrosis found Bone Spur", The Journal of Hiroshima Dental Association, 1995, volume 23, pages 43-47. The problem of the temporomandibular joints is called the TMJ (temporomandibular joint) arthrosis. It is said that the improvement of the TMJ arthrosis is accompanied by disappearance (improvement) of the systemic disorder. It is especially difficult to cure the TMJ arthrosis due to mental causes. Some patients have suffered from pains of temporomandibular joints for a few to more than ten years, and the pains have bothered the patients and changed their lives.

Some patients complained of the systemic disorders although they were not diagnosed with the TMJ arthrosis. Having studied these causes, the present applicant found out that the difficulty of the lateral movement of a lower jaw is significantly associated with the systemic disorders.

Because it was not proved that the lateral movement of the lower jaw is associated with the systemic disorder, it has not been an issue to assist a user to laterally move the lower jaw in a smooth manner so as to improve the systemic disorder.

### SUMMARY OF THE INVENTION

The present invention has the object to provide an assisting device for practicing lateral movement of a lower jaw to assist a user to laterally move the lower jaw in a smooth manner.

According to one aspect of the present invention, there is provided an assisting device for practicing lateral movement of a lower jaw to move a cheek-side cusp of a lower molar to a cheek-side surface of an upper molar. The assisting device comprises an occlusion portion, which is placed in an oral cavity of a user and is occluded by an upper central incisor and a lower central incisor, and a handle connected with the occlusion portion. The occlusion portion includes an upper tooth contact surface to be in contact with an incisal edge of the upper central incisor, and a lower tooth contact surface to be in contact with an incisal edge of the lower central incisor. The lower tooth contact surface has a flat surface. The occlusion portion provides a space between the incisal edge of the upper central incisor and the incisal edge of the lower central incisor. The upper tooth contact surface and the lower tooth contact surface respectively cover entire movement ranges of the incisal edge of the upper central incisor and the incisal edge of the lower central incisor when the lateral movement of the lower jaw is being practiced.

Preferably, the upper tooth contact surface and the lower tooth contact surface respectively extend to positions where an incisal edge of a tooth other than the upper central incisor and an incisal edge of a tooth other than the lower central incisor are in contact with the upper tooth contact surface and the lower tooth contact surface.

Further, the occlusion portion is preferably a flat plate having a horseshoe-shape.

Because the occlusion portion is formed of a flat plate according to the above-mentioned configuration, it is possible to assist the user, whose teeth are misaligned due to loss of teeth or the like, to practice the lateral movement of the lower jaw smoothly.

According to another aspect of the present invention, there is provided an assisting device for practicing lateral movement of a lower jaw to move a cheek-side cusp of a lower molar to a cheek-side surface of an upper molar. The assisting device comprises the occlusion portion to be placed in an oral cavity of a user and attached on at least one of an upper central incisor and a lower central incisor. The occlusion portion is in contact with an incisal edge of an opposed tooth and provides a space between an incisal edge of the upper central incisor and an incisal edge of the lower central incisor. A contact surface to be in contact with the incisal edge of the opposed tooth has a flat surface.

According to the present invention, it is possible to assist the user to be able to laterally move the lower jaw in a smooth manner, thereby resolving the difficulty of the lateral movement of the lower jaw and improving the systemic disorder.

### BRIEF DESCRIPTION OF THE DRAWINGS

The object and features of the present invention will become more readily apparent from the following detailed description taken in conjunction with the accompanying drawings in which:
Fig. 1 is a top view of an assisting device for practicing lateral movement of a lower jaw according to the first embodiment of the present invention;
Fig. 2 is a perspective view to illustrate the relation between the assisting device of the first embedment and the teeth;
Fig. 3 is a front view to illustrate how to use the assisting device of the first embodiment;
Fig. 4 is a top view of an assisting device for practicing lateral movement of a lower jaw according to the second embodiment;
Fig. 5 is a top view of an assisting device for practicing lateral movement of a lower jaw according to the third embodiment;
Fig. 6 is a top view of an assisting device for practicing lateral movement of a lower jaw according to the fourth embodiment;
Fig. 7 is a perspective view to illustrate the user having on an assisting device for practicing lateral movement of a lower jaw according to the fifth embodiment;
Fig. 8 is a perspective view to illustrate the user having on a assisting device for practicing lateral movement of a lower jaw according to the sixth embodiment; and
Fig. 9 shows vertical cross-sections of the right side of an oral cavity to explain lateral movement of a lower jaw.

### DETAILED DESCRIPTION OF THE INVENTION

Hereinafter, a detail description will be given on embodiments of the present invention with reference to the attached drawings.

The applicant of the present invention found out that the difficulty of the lateral movement of a lower jaw is significantly associated with systemic disorders, having studied causes of the systemic disorders, and based on this finding the applicant has completed the present invention.

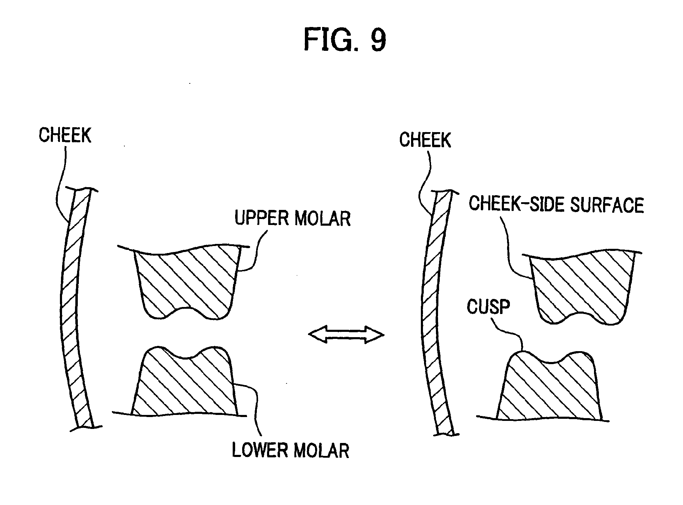

It should be noted that "lateral movement of a lower jaw" means a movement of a lower jaw (teeth grinding) in the right-to-left direction with upper and lower anterior teeth or molars in contact with each other. For example, the lateral movement can be described as that a cheek-side cusp of a lower molar is moved to a cheek-side surface of an upper molar, as shown in Fig. 9.

### <First embodiment>

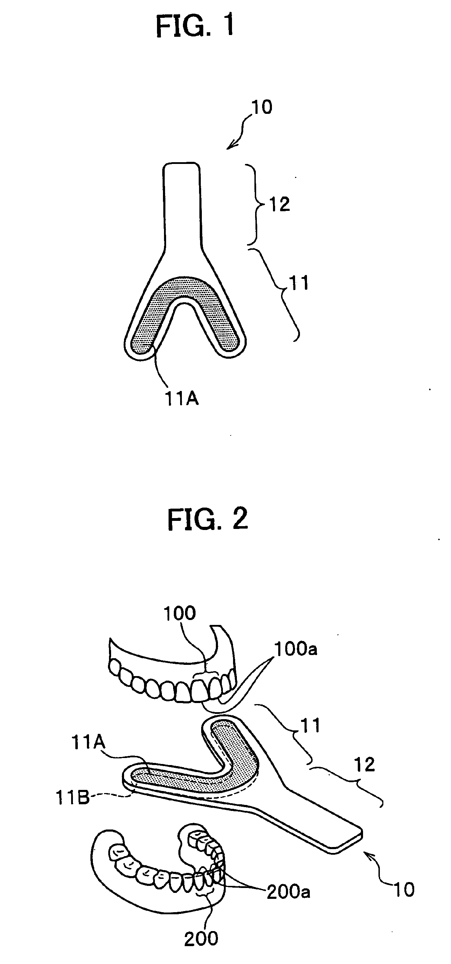

Fig. 1 is a top view of an assisting device for practicing lateral movement of a lower jaw according to the first embodiment of the present invention. As shown in Fig. 1, an assisting device 10 for practicing lateral movement of a lower jaw according to the first embodiment is a flat plate member having a Y-shape as viewed from above. The assisting device 10 includes an occlusion portion 11, which is placed in an oral cavity of a user and is occluded by upper and lower teeth, and a handle 12 to be held by the user. The occlusion portion 11 has a horseshoe-shape as viewed from above.

Preferably, the assisting device 10 is made of a light material so that the user can easily hold the assisting device 10 with one hand. The occlusion portion 11 of the assisting device 10 is made of a material that is nontoxic to a human body because the occlusion portion 11 is placed in the oral cavity of the user. Furthermore, the occlusion portion 11 is preferably made of a hard material that withstands the abrasion, because the occlusion portion 11 is abraded by the lower teeth upon the lateral movement of the lower jaw. Specifically, materials of the assisting device 10 preferably include a plastic or a metal, which satisfies the above-mentioned requirements.

### « The occlusion portion »

Fig. 2 is a perspective view to illustrate the relation between the assisting device of the first embodiment and the teeth. As shown in Fig. 2, the occlusion portion 11 includes an upper tooth contact surface 11A and a lower tooth contact surface 11B. Incisal edges 100a of upper central incisors 100 and incisal edges 200a of lower central incisors 200 are in contact with the upper tooth contact surface 11A and the lower tooth contact surface 11B, respectively, when the occlusion portion 11 is placed in the oral cavity of the user and occluded by the upper central incisors 100 and the lower central incisors 200. In Fig. 2, the lower tooth contact surface 11B is arranged on the lower side of the occlusion portion 11 and is opposed to the upper tooth contact surface 11A.

By the above-mentioned configuration of the occlusion portion 11, a space can be provided between the incisal edges 100a of the upper central incisors 100 and the incisal edges 200a of the lower central incisors 200 when the occlusion portion 11 is placed in the oral cavity of the user and occluded by the upper central incisors 100 and the lower central incisors 200.

The space between the incisal edges 100a and the incisal edges 200a enables the user to laterally move the lower jaw in a smooth manner.

The space may be easily varied by changing the thickness or shape of the assisting device 10, and the space may be appropriately determined such that the user can laterally move the lower jaw in an easy manner.

Because the occlusion portion 11 is formed of a flat plate, it is possible to assist the user, whose teeth are misaligned due to loss of teeth or the like, to laterally move the lower jaw in a smooth manner.

It is preferable that the lower tooth contact surface 11B has a flat surface so that the lower central incisors 200 can easily slide in contact with the lower tooth contact surface 11B upon the lateral movement of the lower jaw.

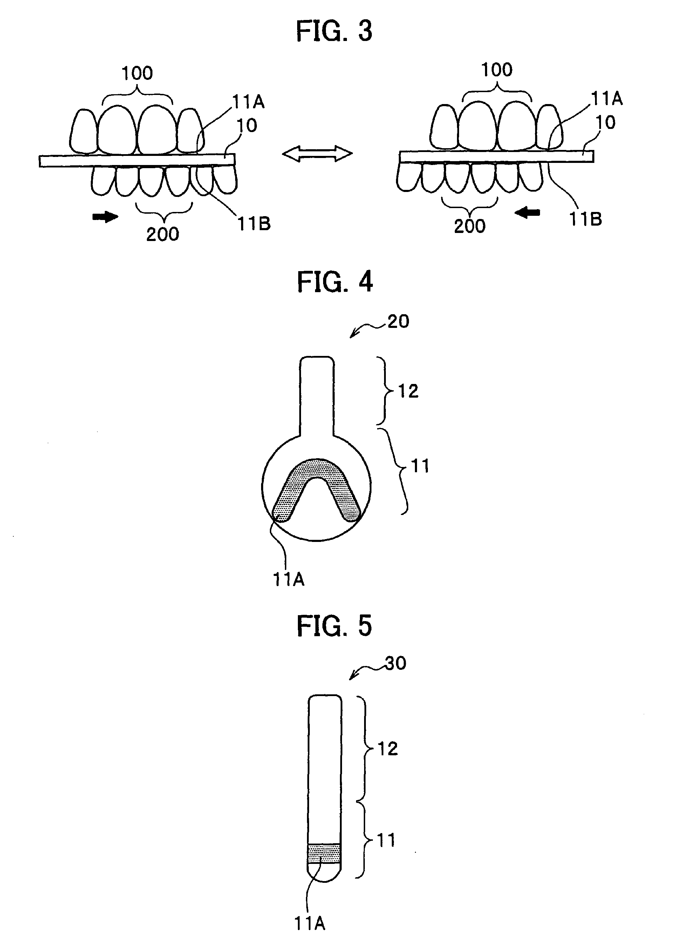

Preferably, the upper tooth contact surface 11A and the lower tooth contact surface 11B have the widths to cover entire movement ranges of the incisal edges 100a of the upper central incisors 100 and the incisal edges 200a of the lower central incisors 200, which are in contact with the upper tooth contact surface 11A and the lower tooth contact surface 11B, respectively. In other words, the upper and lower tooth contact surfaces 11A, 11B preferably have the widths such that the incisal edges 100a and the incisal edges 200a can be in contact with the upper and lower tooth contact surfaces 11A, 11B in both cases where the lower jaw slides to the leftmost position (see Fig. 3) and to the rightmost position (see Fig. 3).

As mentioned above, at least the upper central incisors 100 and the lower central incisors 200 are in contact with the upper tooth contact surface 11A and the lower tooth contact surface 11B in order to provide the space between the incisal edges 100a and the incisal edges 200a. However, anterior teeth or molars other than the upper and lower central incisors 100, 200 may be in contact with the upper and lower tooth contact surfaces 11A, 11B.

According to the above-mentioned configuration of the upper and lower tooth contact surfaces 11A, 11B, it is possible to hold the assisting device 10 for practicing lateral movement of the lower jaw in the oral cavity in a stable manner.

The upper and lower tooth contact surfaces 11A, 11B and the occlusion portion 11 may be integrally formed, or may be made of different materials. The entire upper surface of the occlusion portion 11 may be used as the upper tooth contact surface 11A, and the entire lower surface of the occlusion portion 11 may be used as the lower tooth contact surface 11B.

### « The handle »

As shown in Figs. 1 and 2, the handle 12 is connected with the occlusion portion 11. The user holds the handle 12 to securely place the occlusion portion 11 in the oral cavity.

In this embodiment, the handle 12 is the flat plate member and is integrally formed with the occlusion portion 11, but the handle 12 is not limited to this configuration as long as the user can hold the handle 12. For example, the handle 12 may have a cylindrical or rectangular shape. Further, the occlusion portion 11 and the handle 12 may be separately formed and connected to each other by a commonly used connecting device, such as welding or a spring.

### (How to use the assisting device)

With reference to Figs. 2 and 3, a description will be given on how to use the assisting device for practicing lateral movement of the lower jaw according to the first embodiment of the present invention.

Fig. 3 is a front view to illustrate how to use the assisting device for practicing lateral movement of the lower jaw according to the first embodiment.

Firstly, the user holds the handle 12 of the assisting device 10 and horizontally places the occlusion portion 11 in the oral cavity of the user.

Next, the user bites the occlusion portion 11 so that the incisal edges 100a of the upper central incisors 100 and the incisal edges 200a of the lower central incisors 200 are in contact with the upper tooth contact surface 11A and the lower tooth contact surface 11B, respectively.

Then, the user keeps biting the occlusion portion 11 and slides the lower central incisors 200 along the lower tooth contact surface 11B so as to laterally move the lower jaw.

With reference to Figs. 4 to 8, a description will be given on assisting devices for practicing lateral movement of a lower jaw according to the second to sixth embodiments of the present invention. A description will be given on more characteristic configurations of the second to sixth embodiments compared with the configuration of the first embodiment, but a description on the same configuration as that of the first embodiment will be omitted.

### <Second embodiment>

Fig. 4 is a top view of an assisting device for practicing lateral movement of a lower jaw according to the second embodiment. As shown in Fig. 4, an assisting device 20 for practicing lateral movement of a lower jaw according to the second embodiment includes the occlusion portion 11 that is formed into a circle as viewed from above. The occlusion portion 11 includes the upper tooth contact surface 11A having a horseshoe shape. Although not shown in Fig. 4, the lower tooth contact surface 11B is arranged on the lower side of the occlusion portion 11. and is opposed to the upper tooth contact surface 11A.

The assisting device 20 as configured above is suitable when not only the upper and lower central incisors 100, 200 but also other anterior teeth or molars are in contact with the upper and lower tooth contact surfaces 11A, 11B.

### <Third embodiment>

Fig. 5 is a top view of an assisting device for practicing lateral movement of a lower jaw according to the third embodiment. As shown in Fig. 5, an assisting device 30 for practicing lateral movement of a lower jaw according to the third embodiment is a flat plate member having an I-shape as viewed from above. The occlusion portion 11 is the end portion of the assisting device 30 and has the upper tooth contact surface 11A. Although not shown in Fig. 5, the lower tooth contact surface 11B is arranged on the lower side of the occlusion portion 11 and is opposed to the upper tooth contact surface 11A.

The assisting device 30 as configured above is suitable when only the upper and lower central incisors 100, 200 are in contact with the upper and lower tooth contact surfaces 11A, 11B.

### <Fourth embodiment>

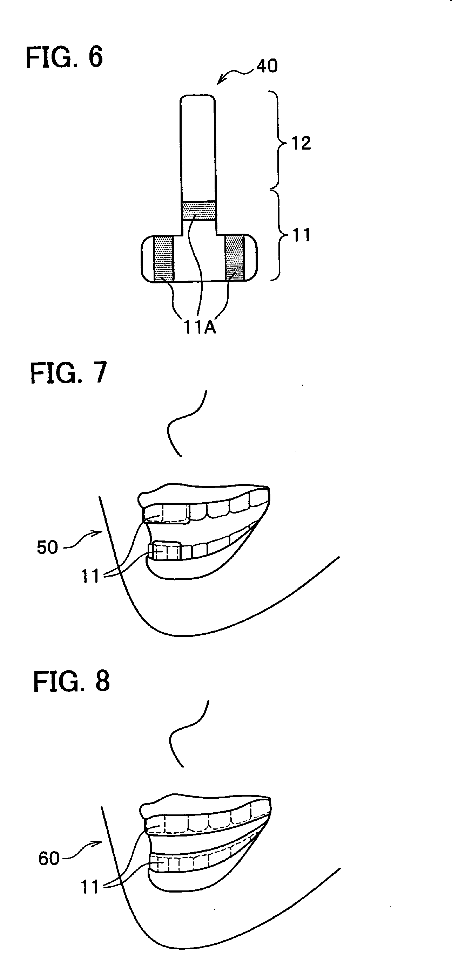

Fig. 6 is a top view of an assisting device for practicing lateral movement of a lower jaw according to the fourth embodiment. As shown in Fig. 6, an assisting device 40 for practicing lateral movement of a lower jaw according to the fourth embodiment is a flat plate member having a T-shape as viewed from above. The occlusion portion 11 is the end portion of the assisting device 40 and has the upper tooth contact surface 11A. Although not shown in Fig. 6, the lower tooth contact surface 11 B is arranged on the lower side of the occlusion portion 11 and is opposed to the upper tooth contact surface 11A.

The assisting device 40 as configured above is suitable when not only the upper and lower central incisors 100, 200 but also other anterior teeth or molars are in contact with the upper and lower tooth contact surfaces 11A, 11B.

### <Fifth embodiment>

Fig. 7 is a perspective view to illustrate the user having on an assisting device for practicing lateral movement of a lower jaw according to the fifth embodiment. As shown in Fig. 7, an assisting device 50 for practicing lateral movement of a lower jaw according to the fifth embodiment includes the occlusion portion 11 and does not include the handle 12 as shown in the first to fourth embodiments. The occlusion portions 11 are formed into a U-shape in cross section and are attached on the upper and lower central incisors 100, 200. The occlusion portion 11 is designed to be in contact with incisal edges of opposed teeth. A contact surface, which the incisal edges of the opposed teeth are in contact with, of the attached occlusion portion 11 has a flat surface. The occlusion portion 11 can provide a space between the incisal edges 100a of the upper central incisors 100 and the incisal edges 200a of the lower central incisors 200.

The assisting device 50 as configured above is suitable when only the upper and lower central incisors 100, 200 are in contact with the occlusion portion 11.

### <Sixth embodiment>

Fig. 8 is a perspective view to illustrate the user having on an assisting device for practicing lateral movement of a lower jaw according to the sixth embodiment. As shown in Fig. 8, an assisting device 60 for practicing lateral movement of a lower jaw according to the sixth embodiment includes the occlusion portion 11 and does not include the handle 12 as shown in the first to fourth embodiments. The occlusion portions 11 are formed into a U-shape in cross section and are attached on the entire upper and lower teeth. The occlusion portion 11 is designed to be in contact with incisal edges of opposed teeth. A contact surface, which the incisal edges of the opposed teeth are in contact with, of the attached occlusion portion 11 has a flat surface. The occlusion portion 11 can provide a space between the incisal edges 100a of the upper central incisors 100 and the incisal edges 200a of the lower central incisors 200.

The assisting device 60 as configured above is suitable when not only the upper and lower central incisors 100, 200 but also other anterior teeth or molars are in contact with the occlusion portion 11.

### (Example)

Hereinafter, a description will be given on a specific example of the present invention.

### « Subject and method of study »

A study was conducted at the Miyama dental office (the director is the present applicant) from April 1, 1999 to November 30, 2005. The 101 patients studied were selected from the patients who were diagnosed with the difficulty of the lateral movement of the lower jaw and were not diagnosed with the TMJ arthrosis. When the 101 patients were questioned on their systemic disorders, 98 out of the 101 patients complained of some systemic disorders, and 3 patients did not complain of any systemic disorders.

The 98 patients, who complained of some systemic disorders, were given sufficient explanation on the study and under their agreements the patients were made to practice the lateral movement of the lower jaw by using assisting devices. The assisting devices 10 (see Fig. 1), which preferably assists the user to laterally move the lower jaw, of the first embodiment were used by the patients. As mentioned above, the assisting device 10 of the first embodiment is a flat plate member having a Y-shape as viewed from above. The lateral movement of the lower jaw is practiced in such a way that the occlusion portion 11 is occluded by the upper and lower teeth, and the lower central incisors 200 slide laterally in the right-to-left direction along the lower tooth contact surface 11B. The patients laterally moved the lower jaws in the right-to-left direction approximately 10 times a day, and then nearly every patient showed the improvement to laterally move the lower jaw in a smooth manner after one to twelve months. Further, the patients continually practiced the lateral movement of the lower jaw by the assisting device 10 until the patients showed the improvement in their systemic disorders. In the case of the patients who had the difficulty of the lateral movement of the lower jaw due to restorative materials such as a crown, inlay, or artificial tooth, the restorative materials were appropriately repaired and the lateral movement of the lower jaw was practiced. Thereby, the patients made the improvement to smoothly move the lower jaw.

With reference to Tables 1 to 3, the results of the study according to the present example will be described.

### « Result of the study »

Table 1 lists the systemic disorders of the 101 patients, who were diagnosed with the difficulty of the lateral movement of the lower jaw and were not diagnosed with the TMJ arthrosis, in order of descending number of patients. As shown in Table 1, the most patients suffered from ear problems, such as ear noises that occur with hearing loss, aural fullness, and vertigo by Meniere's disease. Then, the systemic disorders include, in order, depression or nervous breakdown such as anemia, distraction, or indefinite complaint, limb numbness including difficulty walking, automatic nerve imbalance including the inability to have deep sleep or abnormalities of the heart, migraine, rheumatism, stiffness or pain of shoulder and back, lower-back pain, disorder of taste and smell, increase in blood glucose level, and hand and leg tremors by Parkinson's disease.

Thus, it is assumed that the difficulty of the lateral movement of the lower jaw causes the systemic disorders.

Table 2 shows the configuration of the 98 patients who complained of the systemic disorders according to their sexes and ages. As shown in Table 2, the patients included 23 men and 75 women across the whole age range, mostly in their forties to sixties.

Table 3 shows time required for the 98 patients, who complained of the systemic disorders, to cure their systemic disorders by practicing the lateral movement of the lower jaw. As shown in Table 3, 97 out of the 98 patients cured their systemic disorders, and one patient, who complained of the depression, did not cure the systemic disorder. Further, 90 out of the 97 patients cured their systemic disorders within one year.

**[Table 1]**

| <Systemic disorders complained of by the patients who have the difficulty of the lateral movement of the lower jaw> | | |
|---|---|---|
| Systemic disorders | Number of patients | Total number |
| Ear problems (including the patient being treated for Meniere's disease) | 26 | |
| Depression or nervous breakdown | 15 | |
| Limb numbness including difficulty walking | 14 | |
| Automatic nerve imbalance | 13 | |
| Migraine | 10 | |
| Rheumatism | 6 | |
| Stiffness or pain of shoulder and back | 5 | |
| Lower-back pain | 4 | |
| Disorder of taste and smell | 3 | |
| Increase in blood glucose level | 1 | |
| Hand and leg tremors by Parkinson's disease | 1 | 98 |
| No systemic disorders | 3 | 101 |

**[Table 2]**

| <The configuration of the patients who complained of the systemic disorders according to their sexes and ages > | | | |
|---|---|---|---|
| Ages | Men | Women | Total number according to ages |
| From more than 10 to less than 20 | 1 | 0 | 1 |
| From more than 20 to less than 30 | 1 | 4 | 5 |
| From more than 30 to less than 40 | 1 | 8 | 9 |
| From more than 40 to less than 50 | 4 | 17 | 21 |
| From more than 50 to less than 60 | 7 | 26 | 33 |
| From more than 60 to less than 70 | 7 | 11 | 18 |
| From more than 70 to less than 80 | 1 | 9 | 10 |
| From more than 80 to less than 90 | 1 | 0 | 1 |
| Total number according to sexes | 23 | 75 | Total number: 98 |

**[Table 3]**

| <Time required for the patients to cure their systemic disorders> | | |
|---|---|---|
| Time | Number of patients | Total number |
| Within one month | 3 | |
| Within two months | 9 | |
| Within three months | 30 | |
| Within six months | 26 | |
| Within one year | 22 | |
| More than one year | 7 | |
| No cure is made | 1 | 98 |

Thus, the above-mentioned example shows that the assisting device for practicing lateral movement of the lower jaw according to the present invention can assist the patients having the difficulty of the lateral movement of the lower jaw to laterally move the lower jaw, and the systemic disorders of the patients can be improved in accordance with the improvement of the lateral movement of the lower jaws.

Most of the patients feel that it is natural to have various systemic disorders such as a symptom of menopause when they get old, as if a tree dies as time goes by, and they do not think that they can cure the systemic disorders. Some patient suffered from the systemic disorder for 30 years, which is half of his or her life, or others said that the systemic disorder ruined their lives.

It should be emphasized that even such patients recovered the smooth lateral movement of the lower jaw and improved their systemic disorders by the assisting device for practicing lateral movement of the lower jaw.

It is to be understood that the present invention is not limited to the above-mentioned configurations, and any modification and changes may be made without departing from the scope of the present invention.

## Claims

1. An assisting device for practicing lateral movement of a lower jaw to move a cheek-side cusp of a lower molar to a cheek-side surface of an upper molar, the assisting device comprising:
an occlusion portion to be placed in an oral cavity of a user and occluded by an upper central incisor and a lower central incisor,
the occlusion portion including:
an upper tooth contact surface to be in contact with an incisal edge of the upper central incisor; and
a lower tooth contact surface to be in contact with an incisal edge of the lower central incisor, the lower tooth contact surface having a flat surface,
the occlusion portion providing a space between the incisal edge of the upper central incisor and the incisal edge of the lower central incisor, and a handle connected with the occlusion portion,
wherein the upper tooth contact surface and the lower tooth contact surface cover entire movement ranges of the incisal edge of the upper central incisor and the incisal edge of the lower central incisor, respectively, when the lateral movement of the lower jaw is being practiced.

2. The assisting device according to claim 1,
wherein the upper tooth contact surface and the lower tooth contact surface respectively extend to positions where an incisal edge of a tooth other than the upper central incisor and an incisal edge of a tooth other than the lower central incisor are in contact with the upper tooth contact surface and the lower tooth contact surface.

3. The assisting device according to claim 2,
wherein the occlusion portion is a flat plate having a horseshoe-shape.

4. An assisting device for practicing lateral movement of a lower jaw to move a cheek-side cusp of a lower molar to a cheek-side surface of an upper molar, the assisting device comprising:
an occlusion portion to be placed in an oral cavity of a user and attached on at least one of an upper central incisor and a lower central incisor, the occlusion portion being in contact with an incisal edge of an opposed tooth and providing a space between an incisal edge of the upper central incisor and an incisal edge of the lower central incisor,
wherein the occlusion portion include a contact surface to be in contact with the incisal edge of the opposed tooth, the contact surface having a flat surface.
